# EUROPÄISCHE PATENTSCHRIFT

(11) **EP 0 815 451 B1**
(45) Veröffentlichungstag und Bekanntmachung des Hinweises auf die Patenterteilung: **24.10.2001**
(21) Anmeldenummer: 96908049.8
(22) Anmeldetag: 15.03.1996
(51) Int. Cl.: G01N 33/68

(54) **VERFAHREN ZUR UNTERSUCHUNG VON HAUSSTAUB**
METHOD OF EXAMINING HOUSEHOLD DUST
PROCEDE D'ANALYSE DE LA POUSSIERE DOMESTIQUE

(30) Priorität: 24.03.1995 DE 19510810; 18.05.1995 DE 19518287
(43) Veröffentlichungstag der Anmeldung: 07.01.1998
(73) Patentinhaber: VORWERK & CO. INTERHOLDING GmbH, 42275 Wuppertal (DE)
(72) Erfinder: SINCLAIR, Norman, D-48341 Altenberge (DE); SAUER, Ralf, D-45130 Essen (DE); POCH, Heike, D-42855 Remscheid (DE); VÖLKER, Wolfgang, D-49549 Ladbergen (DE)
(74) Vertreter: Müller, Enno, Dipl.-Ing.
(86) Internationale Anmeldenummer: EP9601128
(87) Internationale Veröffentlichungsnummer: WO9630764

(56) Entgegenhaltungen:
- EP-A- 0 345 582
- CLIN. CHEM., Bd. 31, Nr. 8, 1986, WASHINGTON DC, Seiten 1551-1554, XP002008803 WATANABE, N. ET AL.: "Urinary protein as measured with a pyrogallol red-molybdate complex, manually and in a Hitachi 726 automated analyzer"
- PATENT ABSTRACTS OF JAPAN vol. 011, no. 177 (P-583), 6.Juni 1987 & JP,A,62 006170 (WAKO PURE CHEM IND LTD), 13.Januar 1987,
- PATENT ABSTRACTS OF JAPAN vol. 010, no. 358 (P-522), 2.Dezember 1986 & JP,A,61 155757 (WAKO PURE CHEM IND LTD), 15.Juli 1986,

## Beschreibung

Die Erfindung betrifft zunächst ein Verfahren zur Untersuchung von Hausstaub im Hinblick auf ein allergische Reaktionen auslösendes Potential.

Desweiteren betrifft die Erfindung auch die Verwendung eines Detektionsmittels zur Abschätzung eines allergische Reaktionen auslösenden Potentials von Hausstaub.

Es ist eine allgemeine Feststellung, daß in großem Umfang und sogar zunehmend Allergien bzw. allergische Reaktionen auftreten. Insbesondere auch in Wohnungen und Haushaltungen vorhandene Verunreinigungen wie Milbenexkremente, Schimmelpilze, Pollen, bestimmte pflanzliche Fragmente von Gräsern und Birken sowie Hautschuppen werden hierfür verantwortlich gemacht. Man versucht, mit bekannten Reinigungstechniken, diese Verunreinigungen zu beseitigen, wobei solche Verunreinigungen dann als Bestandteile des aufgesammelten Hausstaubes anfallen. Es besteht daher ein Bedürfnis, eine wirkungsvolle Sammlung und Entfernung des Hausstaubes überprüfen zu können. Weiter besteht auch ein Bedürfnis, festzustellen, inwieweit der Hausstaub insgesamt, als Maß für ein allergisches Potential, mit den vorstehend angeführten Verunreinigungen versetzt ist. Man kann hieraus auch beispielsweise einen Hinweis gewinnen, nach besonderen Verunreinigungsquellen zu suchen.

Aus der Literaturstelle A. Andersen & J. Roesen: "House dust mite...." ist eine Bestimmung der Proteinkonzentration von Hausstaub-Milben bekannt geworden.

Ausgehend von dem vorbeschriebenen Stand der Technik ist eine technische Problematik der Erfindung darin zu sehen, ein Verfahren zur Untersuchung von Hausstaub anzugeben, welches Rückschlüsse auf ein allergisches Potential des Hausstaubes zuläßt. Weiter wird ein technisches Problem darin gesehen, die Verwendung eines geeigneten Detektionsmittels anzugeben.

Diese aufgezeigte Problematik ist zunächst und im wesentlichen beim Gegenstand des Anspruches 1 gelöst, wobei darauf abgestellt ist, daß der Hausstaub zur Untersuchung mit einem Proteindetektor zur größenordnungsmäßigen Feststellung von in dem Hausstaub enthaltenen proteinhaltigen Bestandteilen versetzt wird. Erfindungsgemäß ist erkannt worden, daß praktisch alle oben aufgeführten allergenen Bestandteile des Hausstaubes aus Proteinen aufgebaut sind bzw. Proteinbestandteile besitzen. Weiter wurde erkannt, daß ein Kenntlichmachen, insbesonders ein anteilsmäßiges Kenntlichmachen dieser proteinhaltigen Bestandteile im Hausstaub zugleich ein Maß für das Potential des betreffenden Hausstaubes ist, allergische Reaktionen auszulösen. In weiterer Einzelheit ist vorgesehen, daß der Proteindetektor ein Farbstoff ist, welcher bevorzugt auf proteinhaltige Bestandteile des Hausstaubes reagiert, insbesondere unter Farbumschlag ausfällt. Bei dieser Ausgestaltung des Verfahrens ist es in einfacher Weise möglich, anhand der Farbintensität den größenordnungsmäßigen Anteil der potentiell allergische Reaktionen auslösenden Bestandteile des Hausstaubes festzustellen. Die sich einstellende Farbintensität, nach Einwirken des Proteindetektors auf den Hausstaub, wird hierzu herangezogen. In weiterer bevorzugter Ausgestaltung des Verfahrens ist vorgesehen, daß als Farbstoff Pyrogallolverwendet wird. Im einzelnen handelt es sich um Pyrogallol-Rot-Mo-Komplex, wobei im weiteren ein Bezug auf "Pyrogallol" dieses einfließt. Die Verwendung eines Farbstoffes und insbesonde des Farbstoffes Pyrogallol allgemein zur Untersuchung von Hausstaub wird auch als - weiterer und unabhängiger - Gegenstand der Anmeldung betrachtet. Bei Pyrogallol ist bezüglich Proteinen bzw. proteinhaltigen Partikeln eine Färbung in einem violetten oder auch blauen Farbton zu beobachten, während der Farbstoff selbst zunächst rötlich ist. Hinsichtlich des Detektors empfiehlt es sich weiter, daß der Farbstoff in einer Flüssigkeit gelöst ist. Diese Flüssigkeit kann als Bestandteile insbesondere ein Puffer-Mittel und bevorzugt Stabilisierungszusätze aufweisen. Das Puffer-Mittel dient dazu, zu verhindern, daß bereits PH-Wert-Änderungen der Flüssigkeit zu einer Ausfällung oder Farbintensitätsänderung des Farbstoffes führen. In darüber hinaus weiter bevorzugter Ausgestaltung, jedenfalls im Hinblick auf eine Anwendung, bei welcher eine Kleinstmenge von Hausstaub untersucht werden soll, ist vorgesehen, daß die Flüssigkeit ein Lösungsmittel enthält. Bevorzugtes Lösungsmittel ist weiterer Einzelheit vergällter Alkohol. Beispielsweise 50%iger vergällter Alkohol oder 0,2 mol HCL-Glycerin Puffer gemischt im Verhältnis 1:1 mit 96%igem vergälltem Alkohol. Der Alkohol hat zugleich auch die vorteilhafte kombinative Wirkung, ein Aufschließen der fraglichen Bestandteile zu bewirken. Eventuelle Fettüberlagerungen oder Fettbestandsteile können gelöst werden.

Gegenstand der Erfindung ist des weiteren die Verwendung eines Detektionsmittels zur Abschätzung eines allergische Reaktionen auslösenden Protentials von Hausstaub. Das hierbei verwendete Detektionsmittel zeichnet sich dadurch aus, einen Detektionsbestandteil aufzuweisen, welcher einen Proteinanteil in dem Hausstaub kenntlich macht.

Bevorzugt ist dieser Detektionsbestandteil ein Farbstoff, welcher auf proteinhaltige Bestandteile des Hausstaubes bevorzugt unter Farbumschlag reagiert bzw. ausfällt, wobei zu der größenordnungsmäßigen Bestimmung des Anteiles dieser Bestandsteile eine sich einstellende Farbintensität heranziehbar ist. Dieser Farbstoff kann insbesondere ein Pyrogallol-Rot-Mo-Komplex sein. Das Detektionsmittel kann eine Flüssigkeit sein, in welcher der Farbstoff gelöst ist. Diese Flüssigkeit kann als Bestanteile in Puffer-Mittel und bevorzugt Stabilisierungszusätze aufweisen. Darüber hinaus kann die Flüssigkeit ein Lösungsmittel enthalten. Auf obige Ausführungen wird auch verwiesen.

Ein Ausführungsbeispiel einer Untersuchungsflüssigkeit bzw. eines Detektionsmittels wie es vorstehend beschrieben ist, setzt sich wie folgt zusammen:
1. Glycin-HCL als Pufferbestandteil in der Konzentration 0,2 mol/l und dem PH-Wert 1,9, gemischt im Verhältnis 1:1 mit 96%igen Methyläthylketon-vergälltem Alkohol.
2. Den Bestandteilen 0,2 g/l Pyrogallol Rot
   - 0,3 g/l Ammonium Molybdat Tetrahydrat
   - 1,0 g/l Natrium Oxalat
   - 1,2 g/l (L+)-Weinsäure
3. Verdünnung im Verhältnis 1:10 mit 0,2 mol HCH-Glycin Puffer gemischt im Verhältnis 1:1 mit 96%igem vergälltem Alkohol.

Punkt 3 wird dann angewendet, wenn die Flüssigkeit als Reagenz zur Färbung von Hausstaub in einer Suspension verwendet werden soll. Ohne den Zusatz gemäß Punkt 3 kann die Flüssigkeit auch als konzentrierte Stammlösung zur Direktfärbung, beispielsweise von Filtern eines Staubsaugers, verwendet werden.

Im Anwendungsfall werden beispielsweise 2 bis 4 Tropfen der konzentrierten Pyrogallol-Rot-Molybdän-Stammlösung auf ca. 1 cm ² großes muldenartig geformtes Filterstück, das aus Polypropylen bestehen kann, aufgetropft. Das Filterstück sollte gut durchtränkt, aber nicht mit überschüssiger Lösung bedeckt sein. Je nach Umgebungstemperatur und Menge und Art des gebundenen Feinstaubes färbt sich das Staubmaterial im Filter je nach Proteinbelastung nach maximal fünf Minuten blau-violett.

Ein anderes Anwendungsbeispiel betrifft die 1:10 mit vergälltem 50%igen Alkohol verdünnte Flüssigkeit, (vergleiche beispielsweise Punkt 3 obigen Ausführungsbeispieles). Etwa 1 ml dieser Pyrogallol-Rot-Molybdän-Komplexlösung wird mit einer "Messerspitze" Hausstaub versetzt. Durch Schütteln der beispielsweise in einem Reagenzglas enthaltenen Flüssigkeit in der wärmenden Hand wird der Farbumschlag nach blau-violett beschleunigt. Eine Violettfärbung ist bei üblichem Hausstaub bereits nach 1 bis 2 Minuten festzustellen.

Von derart gekennzeichneten Partikeln aus dem Hausstaub können des weiteren auch noch mikroskopische Analysen vorgenommen werden. Die Flüssigkeit bzw. der Farbstoff beeinträchtigen solche weiterführende Analysen nicht wesentlich. Dies betrifft sowohl Partikel, die in Filtern gefärbt sind wie Hausstaubsuspensionen, die mit der verdünnten Flüssigkeit gefärbt worden sind.

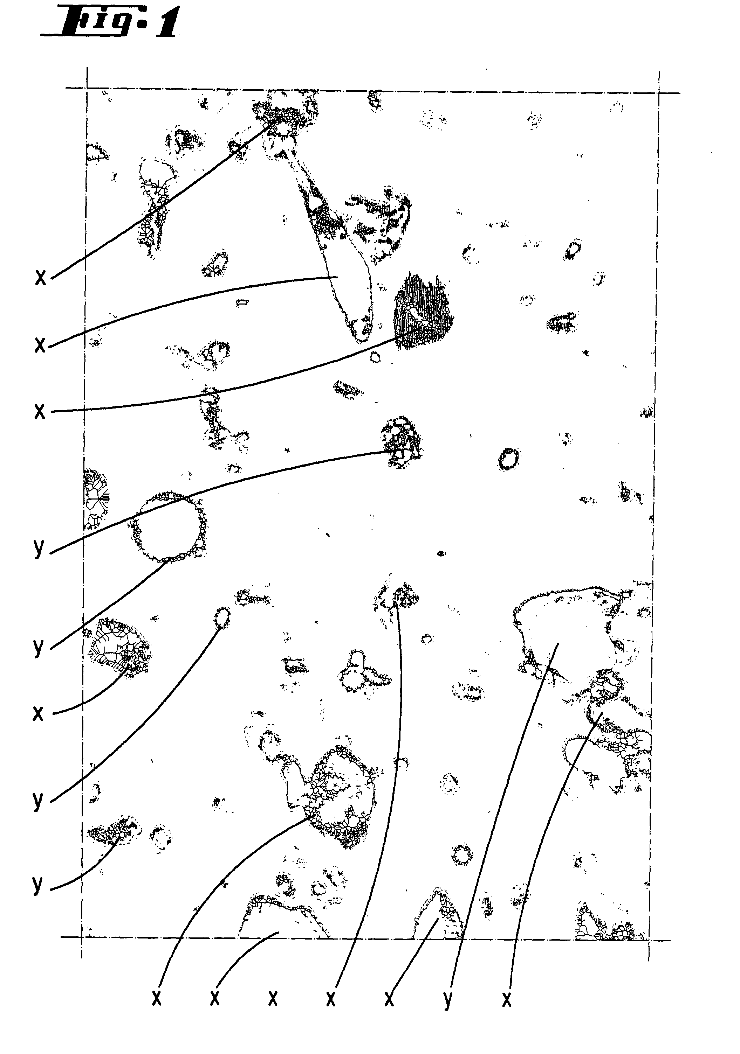

Als Anlage ist als einzige Figur eine Kopie einer hierbei erhaltbaren Mikroaufnahme dargestellt. Die mit x gekennzeichneten Partikel zeigen bei farblicher Wiedergabe eine deutliche blau-bzw. violett-Färbung, während die mit y gekennzeichneten Partikel eine sonstige Färbung oder im wesentlichen keine Färbung aufweisen.
Partikel, die keine Färbung aufweisen, sind beispielsweise Sandkörner oder auch Zuckerkörner.

Die in der vorstehenden Beschreibung, der Zeichnung und den Ansprüchen offenbarten Merkmale können sowohl einzeln als auch in beliebiger Kombination für die Verwirklichung der Erfindung von Bedeutung sein. Alle offenbarten Merkmale sind erfindungswesentlich. In die Offenbarung der Anmeldung wird hiermit auch der Offenbarungsinhalt der zugehörigen/beigefügten Prioritätsunterlagen (Abschrift der Voranmeldung) voll inhaltlich mit einbezogen.

## Patentansprüche

1. Verfahren zur Untersuchung von Hausstaub im Hinblick auf ein allergische Reaktionen auslösendes Potential, **gekennzeichnet durch** ein Versetzen des Hausstaubes mit einem Proteindetektor zur größenordnungsmäßigen Festellung von in dem Hausstaub enthaltenen proteinhaltigen Bestandteilen.

2. Verfahren nach Anspruch 1, **dadurch gekennzeichnet, daß** der Proteindetektor ein Farbstoff ist, welcher bevorzugt auf proteinhaltigen Bestandteilen des Hausstaubes unter Farbumschlag ausfällt und daß zu der größenordnungsmäßigen Bestimmung eine sich einstellende Farbintensität herangezogen wird.

3. Verfahren nach einem oder mehreren der vorhergehenden Ansprüche, **dadurch gekennzeichnet, daß** der Farbstoff Pyrogallol ist.

4. Verfahren nach einem oder mehreren der vorhergehenden Ansprüche, **dadurch gekennzeichnet, daß** der Farbstoff in einer Flüssigkeit gelöst ist, welcher als Bestandteile Puffer-Mittel und bevorzugt Stabilisierungszusätze aufweist.

5. Verfahren nach einem oder mehreren der vorhergehenden Ansprüche, **dadurch gekennzeichnet, daß** die Flüssigkeit ein Lösungsmittel enthält.

6. Verfahren nach einem oder mehreren der vorhergehenden Ansprüche, **dadurch gekennzeichnet, daß** das Lösungsmittel ca. 50%iger, vergällter Alkohol ist.

7. Verwendung eines Detektionsmittels mit einem einen Protein-Anteil kenntlich machenden Detektionsbestandteil zur Abschätzung eines allergische Reaktionen auslösenden Potentials von Hausstaub.

8. Verwendung nach Anspruch 7, **dadurch gekennzeichnet, daß** der Detektionsbestandteil ein Farbstoff ist.

9. Verwendung nach einem der vorhergehenden Ansprüche 7 oder 8, **dadurch gekennzeichnet, daß** der Farbstoff Pyrogallol ist.

10. Verwendung nach einem oder mehreren der vorhergehenden Ansprüche 7 bis 9, **dadurch gekennzeichnet, daß** der Farbstoff in einer Flüssigkeit gelöst ist, welche als Bestandteil einen Puffer-Mittel und bevorzugt Stabilisierungszusätze aufweist.

11. Verwendung nach einem oder mehreren der vorhergehenden Ansprüche 7 bis 10, **dadurch gekennzeichnet, daß** die Flüssigkeit ein Lösungsmittel enthält.

12. Verwendung nach einem oder mehreren der vorhergehenden Ansprüche 7 bis 11,**dadurch gekennzeichnet, daß** das Lösungsmittel ca. 50%iger, vergällter Alkohol ist.

## Claims

1. Method for the investigation of house dust with respect to a potential for inducing allergic reactions, **characterized by** mixing the house dust with a protein detector for establishing the level of protein-containing constituents contained in the house dust.

2. Method according to Claim 1, **characterized in that** the protein detector is a dye which preferably precipitates on protein-containing constituents of the house dust with a colour change, and **in that** colour intensity which develops is used for the determination of the level.

3. Method according to one or more of the preceding claims, **characterized in that** the dye is pyrogallol.

4. Method according to one or more of the preceding claims, **characterized in that** the dye is dissolved in a liquid which contains buffer compositions and preferably stabilizing additives as constituents.

5. Method according to one or more of the preceding claims, **characterized in that** the liquid contains a solvent.

6. Method according to one or more of the preceding claims, **characterized in that** the solvent is denatured alcohol of about 50% strength.

7. Use of a detection agent having a detection constituent which indicates a protein content for estimating a potential of house dust for inducing allergic reactions.

8. Use according to Claim 7, **characterized in that** the detection constituent is a dye.

9. Use according to one of the preceding Claims 7 or 8, **characterized in that** the dye is pyrogallol.

10. Use according to one or more of the preceding Claims 7 to 9, **characterized in that** the dye is dissolved in a liquid which contains a buffer composition and preferably stabilizing additives as constituent.

11. Use according to one or more of the preceding Claims 7 to 10, **characterized in that** the liquid contains a solvent.

12. Use according to one or more of the preceding Claims 7 to 11, **characterized in that** the solvent is denatured alcohol of about 50% strength.

## Revendications

1. Un procédé de test de poussière en milieu domestique en vue de déterminer le risque de déclenchement de réactions allergiques, **caractérisé en ce que** un détecteur de protéines s'ajoute à la poussière domestique en vue de déterminer la proportion des constituants protéiniques contenus dans la poussière domestique.

2. Procédé selon la revendication 1, **caractérisé en ce que** le détecteur de protéines est un colorant, qui se fixe préférentiellement sur les constituants protéiniques de la poussière domestique en modifiant leur couleur et **en ce que** l'on exploite l'intensité de couleur qui s'établit pour déterminer leur proportion.

3. Procédé selon au moins l'une des revendications précédentes, **caractérisé en ce que** le colorant est le pyrogallol.

4. Procédé selon au moins l'une des revendications précédentes, **caractérisé en ce que** le colorant est dissous dans un liquide contenant comme constituants des agents-tampons et des additifs de stabilisation.

5. Procédé selon au moins l'une des revendications précédentes, **caractérisé en ce que** le liquide contient un solvant.

6. Procédé selon au moins l'une des revendications précédentes, **caractérisé en ce que** le solvant est de l'alcool dénaturé à environ 50%.

7. Utilisation d'un moyen de détection comprenant un constituant de détection permettant la détermination de la teneur en protéines et l'évaluation d'un risque de déclenchement de réactions allergiques dues à la poussière domestique.

8. Utilisation selon la revendication 7, **caractérisée en ce que** le constituant de détection est un colorant.

9. Utilisation selon la revendication 7 ou 8, **caractérisée en ce que** le colorant est le pyrogallol.

10. Utilisation selon au moins l'une des revendications 7 à 9, **caractérisée en ce que** le colorant est dissous dans un liquide contenant comme constituant un agent de tampon et de préférence des additifs de stabilisation.

11. Utilisation selon au moins l'une des revendications 7 à 10, **caractérisée en ce que** le liquide contient un solvant.

12. Utilisation selon au moins l'une des revendications 7 à 11, **caractérisée en ce que** le solvant est de l'alcool dénaturé à environ 50%.
